(19) Europäisches Patentamt
European Patent Office
Office européen des brevets

(11) **EP 3 166 594 B1**

(12) **EUROPEAN PATENT SPECIFICATION**

(45) Date of publication and mention
of the grant of the patent:
**07.03.2018 Bulletin 2018/10**

(21) Application number: **15744132.0**

(22) Date of filing: **08.07.2015**

(51) Int Cl.:
***A61K 9/14*** *(2006.01)*

(86) International application number:
**PCT/EP2015/065599**

(87) International publication number:
**WO 2016/005447 (14.01.2016 Gazette 2016/02)**

(54) **A PROCESS FOR PREPARING THE INHALATION FORMULATIONS**

VERFAHREN ZUR HERSTELLUNG VON INHALATIONSFORMULIERUNGEN

PROCÉDÉ DE PRÉPARATION DE FORMULATIONS POUR INHALATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **09.07.2014 TR 201408049**

(43) Date of publication of application:
**17.05.2017 Bulletin 2017/20**

(73) Proprietor: **Arven Ilac Sanayi Ve Ticaret A.S.**
**Istanbul 34460 (TR)**

(72) Inventors:
• **TÜRKYILMAZ, Ali**
**34460 Istanbul (TR)**
• **CELIK, Devrim**
**34460 Istanbul (TR)**
• **AKDAS, Özlem**
**34460 Istanbul (TR)**

(74) Representative: **Sevinç, Erkan**
**Istanbul Patent A.S.**
**Plaza-33, Büyükdere Cad. No: 33/16**
**Sisli**
**34381 Istanbul (TR)**

(56) References cited:
**WO-A1-2012/028663**

• **Yahya Rahimpour ET AL: "Lactose engineering for better performance in dry powder inhalers", Advanced pharmaceutical bulletin, 1 January 2012 (2012-01-01), pages 183-187, XP055162111, Iran DOI: 10.5681/apb.2012.028 Retrieved from the Internet: URL:http://www.ncbi.nlm.nih.gov/pubmed/243 12791 [retrieved on 2015-01-15]**
• **Hamed Hamishehkar ET AL: "The Role of Carrier in Dry Powder Inhaler" In: "Recent Advances in Novel Drug Carrier Systems", 31 October 2012 (2012-10-31), InTech, XP055162513, ISBN: 978-9-53-510810-8 DOI: 10.5772/51209, the whole document**
• **, 14 July 2004 (2004-07-14), XP055162511, Retrieved from the Internet: URL:http://www.ncbi.nlm.nih.gov/pmc/articl es/PMC2751242/pdf/12248_2008_Article_6301. pdf [retrieved on 2015-01-15]**

**Description**

**Field of the invention**

The present invention relates to a novel process used for the preparation of dry powder formulations for inhalation.

**Background of the invention**

For treating a number of respiratory diseases such as asthma, chronic obstructive disease (COPD), it is useful to administer the active substance by inhalation. Preferably, the dry powder formulations for the treatment of respiratory diseases are administered through inhalation, since they are directly delivered to the affected sites (airways) in high doses via this route, have a short onset time, and they lack or have minimal systemic side effects. In DPIs, active substances are administered as a powder after formulating them with inert carriers, including lactose, glucose, and mannitol. Compared to other pulmonary drug delivery systems, such as nebulizers and pMDIs, DPIs offer several advantages, including enhanced drug stability (i.e. active substance stability), greater accuracy in dosing, elimination of hand-to-mouth coordination, breath-actuated delivery, and consequently, an overall improvement in patient compliance.

Typically, DPI's contain a dose system, which contains the powder formulation either in bulk supply or quantified into individual doses stored in unit dose compartments, like hard gelatin capsules or blisters. Bulk containers are equipped with a measuring system operated by the patient in order to isolate a single dose from the powder immediately before inhalation.

Dry powder formulations are generally formulated as a powder mixture of coarse carrier and micronized active substance with mass median aerodynamic particle diameters of 1-5 $\mu$m. Only small amount of the micronized active substance particles is needed per single dose to provide desired therapeutic effect. Since the size of the active substance particles is very small, it has very poor flowability and it is very difficult to fill the small amount of active substance particles into unit dose compartments or bulk containers. The poor flowability is also detrimental to the active substance unable to leave the inhaler and remaining adhered to the interior of the inhaler or leaving the inhaler as large agglomerates; agglomerated particles, in turn, cannot reach the bronchiolar and alveolar sites of the lungs. The uncertainty as to the extent of agglomeration of the particles between each actuation of the inhaler and also between inhalers and different batches of particles, leads to poor dose reproducibility as well. Because of their poor flowability and extreme agglomeration tendency, achieving the high dose reproducibility with micronised active substance particles is also difficult.

Successful management of the respiratory diseases such as asthma, chronic obstructive pulmonary disease (COPD) depends on achieving adequate delivery of inhaled active substances to the lung and improving lung deposition. For this purpose, it is needed to prepare the dry powder formulations with high dose reproducibility. High dose reproducibility requires excellent content uniformity and reproducible dose weighing of the powder into the dose system (capsule, blister, bulk container, etc) as well as complete discharge of this dose system by the inspiratory air during inhalation.

Therefore, the aim of the present invention is to provide a process which is used for preparing the homogeneous dry powder formulation with high content uniformity that enables high dose reproducibility to be achieved.

**The Detailed Description of the Invention**

The active substance has to be diluted with suitable carriers to prepare dry powder formulation for inhalation. Carrier particles are used to improve active substance flowability, thus improving dosing accuracy, minimizing the dose variability compared with active substance alone and making them easier to handle during manufacturing operations. Additionally, with the use of carrier particles, active substance particles are emitted from the medicament compartments (capsule, blister, etc.) more readily, hence, complete discharge of the medicament compartments by the inspiratory air during inhalation can be achieved and the inhalation efficiency in terms of emitted dose and fine particle fraction (FPF) increases.

Additionally, moisture uptake can directly affect the flowability of the micronized powders and the force to detach the micronized active substance particles from the carrier surface. It is known that use of an additive such as magnesium stearate, also helps to minimize the influence of penetrating moisture during the storage of said formulation and results in the dry powder formulation to be more stable against the moisture. Thus, the quality of the pharmaceutical formulation remains considerably better than conventional formulations which are free of magnesium stearate even on storage under extreme conditions of temperature and humidity. Therefore, use of magnesium stearate also improves the moisture resistance of the dry powder formulations.

The active substance has to be mixed with carrier and/or additive particles using powder mixture technology for preparing the dry powder formulation. For high dose reproducibility, it is also necessary to perform an efficient mixing process that is used for preparing the dry powder formulation with high content uniformity. Therefore, the process that is used for preparing the dry powder formulation has an important role to produce the homogeneous dry powder formulation in terms of achieving high content uniformity and high dose reproducibility.

It has surprisingly been found that a process for the preparation of the dry powder formulation for inhalation that enable said formulation to be produced with high content uniformity to achieve high dose reproducibility.

The process in accordance with the present invention is used for the preparation of the dry powder formulation comprising an active substance, a pharmaceutically acceptable carrier and magnesium stearate. The process of the invention for preparing the dry powder formulation characterized in that an active substance and an additive are added alternately layer by layer to a suitable mixing apparatus while a pharmaceutically acceptable carrier is continuously metered into the same mixing apparatus concurrently.

In another embodiment of the present invention, the active substance and the additive are divided into the same number of the equal-size portions separately. Then, they are added alternately layer by layer into the mixing apparatus while the pharmaceutically acceptable carrier is continuously metered into the same mixing apparatus concurrently. Additionally, the mixing is proceeding without interruption while the carrier, the magnesium stearate and the active substance is added in accordance with the process of the invention into the mixing apparatus and after the addition of all of the components into the mixing apparatus is completed, the mixing is proceeded some more time. The mixing period, after the addition of all of the components is completed, is between 5 and 210 minutes, preferably 10 and 180 minutes, more preferably 15 and 150 minutes, most preferably 20 and 125 minutes.

Within the scope of the invention, the term "equal-size" means that the amounts of the portions of the any component (the active substance or the additive) are equal to each other and the variability of the amounts of the portions is $\pm 5\%$, preferably $\pm 3\%$, more preferably $\pm 2\%$, most preferably $\pm 1\%$ by weight.

In another embodiment of the present invention, the active substance and the additive are divided into at least two, preferably between 2 and 60, more preferably 2 and 45, most preferably 2 and 30 equal-size portions separately. Additionally, the size of the portions of each component (the additive or the active substance) depends on the total amount of the component. In another words, if the total amount of the additive is more than the total amount of the active substance, when the additive and active substance is divided into the same number of equal size portions separately, the size of the each portion of the additive will also be more than the size of the each portion of the active substance.

In one embodiment of the present invention, the additive that is used in the dry powder formulation prepared by the process of the invention is preferably magnesium stearate.

In another embodiment of the present invention, the components, which are the carrier, the magnesium stearate and the active substance, are added into the suitable mixing apparatus through a suitable screening apparatus. If desired, once the mixing process is finished, the entire powder mixture can be passed through screening apparatus at least one time. According to the invention, the active substance and the additive are added alternately layer by layer into the mixing apparatus. This layered addition is preferably started with the portion of the active substance and it is finished with the portion of the magnesium stearate. This provides an advantage since any residues of the last portion of the active substance still remaining in the screening apparatus can be carried into the mixing apparatus by means of the last portion of the magnesium stearate. The components of the dry powder formulation prepared by the process of the invention are preferably added through a screening apparatus, preferably a sieve, with a mesh size of 0.05 to 3 mm, more preferably 0.1 to 1.0 mm, most preferably 0.1 to 0.5 mm.

According to the present invention, the sieve that is used in the process of the invention is suitable for sieving materials that are used for preparing pharmaceutical formulations.

In another embodiment of the present invention, the components in the process are mixed using any suitable blending apparatus, such as high shear mixer (for example a QMM, PMA or TRV series mixer) or a low shear tumbling mixer (a Turbula mixer). The mixing during the preparation of the dry powder formulation is performed using a high shear mixer or a low shear tumbling mixer, whichever is appropriate, with the speed rate of 2 to 250 rpm, preferably 5 to 100 rpm, more preferably 10 to 60 rpm.

In another embodiment of the present invention, the mixing apparatus in which the components of the dry powder formulations (the pharmaceutically acceptable carrier, the additive and the active substance) is mixed, is preferably a suitable mixing vessel.

In another embodiment of the present invention, the pharmaceutically acceptable carrier contained in the dry powder formulation prepared by the process of the invention is selected from the group comprising lactose, mannitol, glucose, trehalose, cellobiose, sorbitol, maltitol or a combination of two or more of them, for example a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, or lactose and maltitol. According to the present invention, lactose is preferably used as the pharmaceutically acceptable carrier. Lactose used in the process according to the invention is preferably anyhdrous lactose or lactose monohydrate.

According to the invention, "pharmaceutically acceptable" refers to the properties and/or substances which are acceptable to the patient from a pharmacological-toxicological point of view and to the manufacturing pharmaceutical formulation.

The amount of the pharmaceutically acceptable carrier is much more than the total amount of the active substance and the magnesium stearate in the dry powder formulation prepared by the process of the present invention. Therefore, the particle size of the carrier particles is also important for the flowing properties of the dry powder formulation prepared by the process in accordance with the invention. Therefore, the volume median diameter of the pharmaceutically acceptable carrier, preferably lactose, used in the process of the invention, is between 30 μm and 250 μm, for example 35 μm, 40 μm, 45 μm, 50 μm, 55 μm, 60 μm, 65 μm, 70 μm, 75 μm, 80 μm, 85 μm, 90 μm, 95 μm, 100 μm, 105 μm, 110 μm, 115 μm, 120 μm, 125 μm, 130 μm, 135 μm, 140 μm, 145 μm, 150 μm, 155 μm, 160 μm, 165 μm, 170 μm, 175 μm, 180 μm, 185 μm, 190 μm, 195 μm, 200 μm, 205 μm, 210 μm, 215 μm, 220 μm, 225 μm, 230 μm, 235 μm, 240 μm, 245 μm; preferably between 40 μm and 225 μm, for example 43 μm, 48 μm, 57 μm, 64 μm, 76 μm, 82 μm, 93 μm, 106 μm, 119 μm, 121 μm, 133 μm, 142 μm, 151 μm, 165 μm, 173 μm, 186 μm, 192 μm, 203 μm, 207 μm, 211 μm, 216 μm, 218 μm, 222 μm; more preferably between 45 μm and 215 μm, for example 47 μm, 52 μm, 58 μm, 66 μm, 72 μm, 83 μm, 91 μm, 103 μm, 117 μm, 125 μm, 132 μm, 138 μm, 143 μm, 149 μm, 154 μm, 159 μm, 162 μm, 168 μm, 174 μm, 179 μm, 183 μm, 188 μm, 192 μm, 197 μm, 206 μm, 209 μm, 213 μm; most preferably 50 μm and 200 μm, for example 53 μm, 59 μm, 64 μm, 73 μm, 77 μm, 81 μm, 83 μm, 86 μm, 89 μm, 92 μm, 97 μm, 99 μm, 101 μm, 106 μm, 112 μm, 114 μm, 118 μm, 121 μm, 133 μm, 146 μm, 151 μm, 156 μm, 161 μm, 167 μm, 177 μm, 179 μm, 184 μm, 189 μm, 194 μm, 199 μm.

The carrier is present in the dry powder formulation prepared by the process according to the invention in an amount of 70% to 99%, preferably in an amount of 85% to 99%, more preferably in an amount of 90% to 99%, most preferably in an amount of 95% to 99% by weight based on the total amount of the dry powder formulation.

On the other hand, according to the present invention, the pharmaceutically acceptable carrier used in the process of the invention may preferably consist of two fractions each of which has a different particle-size; fine carrier and coarse carrier. The type of the fine carrier can be the same as or different from the type of the coarse carrier: The fine carrier and coarse carrier may constitute a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, or lactose and maltitol. According to present invention, lactose is preferably used as both of the fine carrier and coarse carrier in the process of the present invention. In one embodiment of the present invention, lactose is anyhdrous lactose or lactose monohydrate.

If the dry powder formulation prepared by the process according to the invention comprises fine carrier and coarse carrier (preferably fine lactose and coarse lactose), the fine carrier and coarse carrier are mixed to obtain carrier mixture before they are mixed with the active substance and the additive. Preferably, the fine carrier and the coarse carrier are mixed in such a way that the fine and coarse carrier are added alternately layer by layer starting from the coarse carrier into the mixing apparatus to obtain carrier-mixture.

It is know that the addition of low surface free energy materials such as magnesium stearate, to the carrier-based dry powder formulation increases the aerosolisation efficiency of dry powder formulations, by decreasing the active substance-carrier adhesion and thus facilitating the active substance detachment upon device actuation. Additionally, use of the magnesium stearate in the dry powder formulation prepared by the process of the present invention, also helps to minimize the influence of penetrating moisture during the storage of said formulation and results in said formulation to be more stable against the moisture. Thus, the quality of the pharmaceutical formulation remains considerably better than conventional formulations which are free of the magnesium stearate even on storage under extreme conditions of temperature and humidity. Therefore, use of magnesium stearate also improves the moisture resistance of the dry powder formulations. However, the magnesium stearate is poorly water soluble, its presence in such amount may raise some concerns as to a potential irritation or toxicity of this excipient, part of which can be inhaled by the patient together with the active substance. Therefore, it is important to determine the optimum concentration of the magnesium stearate that enables eliminating or minimizing potential irritation or toxicity of this excipient while getting balanced interparticulate forces between the active substance and the carrier surface which will enable maximum aerosolisation deposition, and minimizing the influence of penetrating moisture during the storage of the formulation. According to the present invention, the optimum total amount of the magnesium stearate is found as less than 1.5% by weight based on the total amount of the dry powder formulation to achieve aforementioned effects at the same time. The preferred total amount of the magnesium stearate contained in the dry powder formulation prepared by the process according to the invention is between 0.02% and 1.0%, for example 0.04%, 0.06%, 0.08%, 0.2%, 0.25%, 0.30%, 0.35%, 0.40%, 0.45%, 0.55%, 0.60%, 0.65%, 0.70%, 0.75%, 0.80%, 0.85%, 0.90%, 0.95%; more preferred total amount of the magnesium stearate contained in the dry powder formulation prepared by the process according to the invention is between 0.05% and 0.75%, for example 0.075%, 0.085%, 0.14%, 0.18%, 0.24%, 0.28%, 0.34%, 0.38%, 0.44%, 0.48%, 0.52%, 0.56%, 0.64%, 0.68%, 0.72%; most preferred total amount of the magnesium stearate contained in the dry powder formulation prepared by the process according to the invention is between 0.10% and 0.50%, for example 0.13%, 0.16%, 0.19%, 0.21%, 0.23%, 0.26%, 0.29%, 0.31%, 0.33%, 0.36%, 0.39%, 0.42%, 0.46%, 0.49%, by weight based on the total amount of the dry powder formulation.

The volume median diameter of the magnesium stearate contained in the dry powder formulation prepared by the process of the invention is between 1 μm and 100 μm, preferably, 1 μm and 50 μm, more preferably, 1 μm and 25 μm, most preferably 1 μm and 15 μm.

The volume median diameter ($Dv_{50}$ or $Dv_{0.5}$) is the median for a volume distribution in such a way that 50% of the volume of the particle diameter is less than the median and 50% of the volume of the particles diameter is more than the median.

The volume median diameter of the pharmaceutically acceptable carrier, the magnesium stearate and the active substance used in the process of the invention for preparing the dry powder formulation are preferably measured by means of a laser diffraction method. More specifically, the volume median diameter of the carrier and the volume median diameter of the magnesium stearate are measured using a dry dispersion method using air as a dispensing agent on a "Malvern Mastersizer 2000 Particle Size Analyzer". On the other hand, the volume median diameter of the active substance is measured using a dry dispersion or a liquid dispersion method, whichever is appropriate, making use of a suitable dispensing agent (air, water, solvent, etc) on a "Malvern Mastersizer 2000 Particle Size Analyzer".

The pharmaceutical acceptable carrier has a large particle size distribution whereas the active substance and the additive have a smaller particle size distribution. The process according to the present invention provide a homogeneous dry powder formulation in terms of content uniformity that is necessary for achieving high dose reproducibility.

Since the micronized particles, such as the magnesium stearate and the active substance, have high surface energy and thus they are highly adhesive and cohesive, they have poor flowability and are prone to form agglomerated particles. According to the invention, in the process for the preparation of the dry powder formulation comprising the magnesium stearate and the active substance, the method of the addition of these components is of great importance for the homogeneity of the formulation. When the pharmaceutically acceptable carrier, the magnesium stearate and the active substance are mixed in accordance with the process of the invention, the magnesium stearate particles are distributed homogeneously over the surface of much larger carrier particles, and since the adhesion and cohesion forces between carrier and active substance are balanced because of this homogeneous distribution of the magnesium stearate, the active substance is also distributed among the dry powder formulation homogeneously when the active substance is mixed with the carrier. Consequently, the process of the invention provides the dry powder formulation with good content uniformity, and this enable reproducible dose weighing of the powder into the dose system (such as capsule, blister, cartridge, etc.) and complete discharge of this dose system by the inspiratory air during inhalation which are necessary for high dose reproducibility.

The dry powder formulation that is prepared using the process of the invention has also a good flowability for inhaler filling. This also allows accurate metering of said dry powder formulation. Therefore, said formulation can be uniformly filled into blisters, capsules or reservoirs suitably used in dry powder inhalers, and thus, any dose inhaled by a patient from the respective blister, capsule, or reservoir during inhalation can be delivered with a high dose accuracy. Having said that, the dry powder formulation with good flow properties also contributes to an almost complete discharge of the powder from the inhaler during inhalation.

The active substance used in the process of the present invention is selected from a group comprising steroids such as alcometasone, beclomethasone, beclomethasone dipropionate, betamethasone, budesonide, ciclesonide, clobetasol, deflazacort, diflucortolone, desoxymethasone, dexamethasone, fludrocortisone, flunisonide, fluocinolone, fluometholone, fluticasone, fluticasone proprionate, fluticasone furoate, hydrocortisone, triamcinolone, nandrolone decanoate, neomycin sulphate, nimexolone, methylprednisolone and prednisolone; bronchodilators such as β2-agonists including vilanterol, vilanterol trifenatate, salbutamol, formoterol, salmeterol, fenoterol, bambuterol, bitolterol, sibenadet, metaproterenol, epinephrine, isoproterenol, pirbuterol, procaterol, terbutaline and isoetharine antimuscarinics including ipratropium and tiotropium, and xanthines including aminophylhne and theophylline; nitrates such as isosorbide mononitrate, isosorbide dinitrate and glyceryl trinitrate; antihistamines such as azelastine, chlorpheniramine, astemizole, cetirizine, cinnarizine, desloratadine, loratadine, hydroxyzine, diphenhydramine, fexofenadine, ketotifen, promethazine, trimeprazme and terfenadine; anti-inflammatory agents such as piroxicam, nedocromil, benzydamine, diclofenac sodium, ketoprofen, ibuprofen, heparinoid, cromoglycate, fasafungine, lodoxamide and p38 MAP kinase inhibitors, anticholinergic agents such as atropine, benzatropme, bipenden, cyclopentolate, oxybutinin, orphenadine, glycopyrromum, glycopyrrolate, procyclidine, propantheline, propiverine, tiotropium, trihexyphenidyl, tropicamide, trospium, ipratropium bromide and oxitropnum bromide; leukotriene receptor antagonists such as montelukast and zafirlukast; pharmaceutically acceptable salts, solvates, enantiomers, racemic mixtures or derivatives of any of the foregoing.

As used herein, the term "active substance" refers to a substance, as a chemical compound or complex that has a measurable beneficial physiological effect on the body, such as a therapeutic effect in treatment and prophylaxis of a disease or disorder, when administered in an effective amount.

The phrase "effective amount" refers to that amount of a substance that produces some desired local or systemic effect at a reasonable benefit/risk ratio applicable to any treatment.

The present invention relates in particular to the process for preparing dry powder formulation containing the active substance in an amount of 0.05 to 2.5%, more preferably present in an amount of 0.05 to 1.5%, most preferably present in an amount of 0.1 to 1.0 % by weight based on the total amount of the dry powder formulation. The volume median diameter of the active substance contained in the dry powder formulation prepared by the process of the invention is between 0.5 $\mu$m and 15 $\mu$m, preferably 1 $\mu$m and 10 $\mu$m, more preferably 1 $\mu$m and 6 $\mu$m, most preferably 1 $\mu$m and 4.5 $\mu$m.

In another embodiment of the present invention, the active substance used in the process for preparing the dry powder formulation is preferably vilanterol or a pharmaceutically acceptable salt thereof, more preferably vilanterol triphenylacetate (i.e. vilanterol trifenatate).

Vilanterol is a LABA (long-acting $\beta_2$-adrenoceptor agonist) with a 24-hour duration of action that is used for the preparation of a medicament in the prophylaxis and treatment of respiratory diseases such as asthma, chronic obstructive pulmonary diseases (COPD), respiratory tract infection and upper respiratory tract disease. It is also known with the chemical name of 4-{(1R)-2-[(6-{2-[(2,6-dichlorobenzyl)oxy]ethoxy}hexyl)amino]-1-hydroxyethyl}-2-(hydroxymethyl)phenol. Vilanterol or pharmaceutically acceptable salts thereof, in particular the acetate, triphenylacetate, $\alpha$-phe-nylcinnamate, 1 -naphthoate and (R)-mandelate salts, are specifically described in WO03/024439A1 as well as the preparation method thereof.

It is necessary to deliver the active substance with 24-hour duration of action, such as vilanterol, to the lungs in effective amount for the treatment to guarantee the maintenance the effect of the active substance during 24-hour duration for success of the once-daily administration of said formulation. Therefore, the process according to the invention is useful to prepare the dry powder formulation comprising vilanterol or a pharmaceutically acceptable salt thereof, preferably vilanterol triphenylacetate, with good content uniformity and thus also providing high dose reproducibility to guarantee the maintenance the effect of the active substance during 24-hour duration upon each inhalation.

More spesifically, in the light of the abovementioned description, the process according to the present invention for the preparation of the dry powder formulation characterized in that vilanterol triphenylacetate and the magnesium stearate are added alternately layer by layer to a suitable mixing apparatus while the lactose is continuously metered into the same mixing apparatus concurrently.

Within the scope of the invention, the emitted dose (ED) is the total mass of the active substance emitted from the device upon the actuation. It does not include the material left inside or on the surfaces of the device. The ED is measured by collecting the total emitted mass from the device in an apparatus frequently identified as a dose uniformity sampling apparatus (DUSA), and recovering this by a validated quantitative wet chemical assay.

Within the scope of the invention, the fine particle dose (FPD) is the total mass of active substance which is emitted from the device upon the actuation which is present in a mass median aerodynamic particle size smaller than a defined limit. This limit is generally taken to be 5 $\mu$m if not expressly stated to be an alternative limit, such as 3 $\mu$m or 1 $\mu$m, etc. The FPD is measured using an impactor or impinger, such as a twin stage impinger (TSI), multi-stage impinger (MSI), Andersen Cascade Impactor or a Next Generation Impactor (NGI). Each impactor or impinger has a pre-determined aerodynamic particle size collection cut points for each stage. The FPD value is obtained by interpretation of the stage-by-stage active substance recovery quantified by a validated quantitative wet chemical assay where either a simple stage cut is used to determine FPD or a more complex mathematical interpolation of the stage-by-stage deposition is used.

The term "mass median aerodynamic diameter" (MMAD) is a measure of the aerodynamic size of a dispersed aerosol particle. The aerodynamic diameter is used to describe an aerosolized particle in terms of its settling behavior, and is the diameter of a unit density sphere having the same settling velocity, generally in air, as the particle in question. The aerodynamic diameter encompasses particle shape, density, and physical size. MMAD refers to the midpoint or median of the aerodynamic particle size distribution of an aerosolized collection of particles determined by Andersen Cascade Impactor (ACI), Next Generation Impactor (NGI), or Marple Miller Impactor at each of the common flow rates. According to the present invention, the mass median aerodynamic particle diameter of the active substance is between 1 and 5 $\mu$m.

The fine particle fraction (FPF) is normally defined as the FPD divided by the ED and expressed as a percentage. Herein, the FPF of ED is referred to as $FPF_{(ED)}$ and is calculated as

$$FPF_{(ED)}=(FPD/ED)\times100\%$$

According to the present invention, the dose reproducibility is measured in terms of relative standard deviation (RSD %) and is in the order of less than % 20, less than % 15, less than % 10, less than % 5, or less than % 3. Therefore, the good content uniformity and the high dose reproducibility achieved by the process of the present invention guarantee the delivery of the active substance to the lungs in efficient amount necessary for the desired treatment of respiratory diseases upon each inhalation.

The dry powder formulation which is obtained by the process according to the present invention can be delivered by any suitable inhalation device that is adapted to administer a controlled amount of such a pharmaceutical formulation in dry powder form to a patient. Suitable inhalation devices may rely upon the aerosolisation energy of the patient's own breath to expel and disperse the dry powder dose. Alternatively, this energy may be provided by an energy source independent of the patient's inhalation effort, such as by impellers, patient/device created pressurized gas sources or physically (e. g. compressed gas) or chemically stored energy sources. Suitable inhalation devices can also be of the reservoir type i.e. where the dose is withdrawn from a storage vessel using a suitably designed dosing device or alternatively, inhalation devices that release active substance from pre-metered units e. g. blisters, cartridges or capsules.

There are various types of dry powder inhalers, for example, reservoir dry powder inhalers, unit-dose dry powder inhalers, pre-metered multi-dose dry powder inhalers, nasal inhalers or insufflators. The dry powder formulation which is obtained by the process according to the present invention may be presented in unit dosage form, for example, be presented in capsules, cartridges, or blisters for use in an inhaler or insufflator.

The dry powder formulation which is obtained by the process according to the present invention is suitable for administration by oral and nasal inhalation.

Packaging of the dry powder formulation which is obtained by the process according to the present invention may be suitable for unit dose or multi-dose delivery. In one embodiment, the dry powder formulation which is obtained by the process according to the present invention suitable for inhaled administration may be incorporated into a plurality of sealed dose containers provided on medicament pack(s) (e.g. blister) mounted inside a suitable inhalation device. The containers may be rupturable, peelable or otherwise openable one-at-a-time and the doses of the dry powder composition administered by inhalation on a mouthpiece of the inhalation device, as known in the art. The medicament pack may take a number of different forms, for instance a disk-shape or an elongate strip.

The dry powder formulation which is obtained by the process according to the present invention may also be provided as a bulk reservoir in an inhalation device, the device then being provided with a metering mechanism for metering a dose of the composition from the reservoir to an inhalation channel where the metered dose is able to be inhaled by a patient inhaling at a mouthpiece of the device.

A further delivery method for the dry powder formulation which is obtained by the process according to the present invention is for metered doses of the formulation to be provided in capsules (one dose per capsule) which are then loaded into an inhalation device, typically by the patient on demand. The device has means to rupture, pierce or otherwise open the capsule so that the dose is able to be entrained into the patient's lung when they inhale at the device mouthpiece.

If the dry powder formulation obtained by the process according to the invention is to be packed into capsules (inhalettes) in accordance with the preferred application mentioned above, the capsules are filled with the amount of from 3 to 30 mg, preferably from 5 to 25 mg, more preferably 10 to 25 mg of the dry powder formulation per capsule. On the other hand, if the dry powder formulation obtained by the process according to the invention is to be packed into blister strip (preferably elongate peelable blister strip) in accordance with the preferred application mentioned above, the blisters are filled with the amount of from 2 to 15 mg, preferably from 3 to 13 mg, more preferably 4 to 12.5 mg of the dry powder formulation per blister. In the case of the active substance being vilanterol, preferably vilanterol triphenylacetate, the capsule or the blister contain between 1 $\mu$g and 100 $\mu$g, preferably between 2 $\mu$g and 75 $\mu$g, more preferably 5 $\mu$g and 50 $\mu$g of vilanterol as free base.

Vilanterol or a pharmaceutically acceptable salt thereof can be used in combination with one or more other therapeutically active substances as the active substance used in the process of the invention. The one or more other therapeutic substance is selected from a group comprising anti-inflammatory agents, anticholinergic agents (particularly a muscarinic ($M_1$, $M_2$, or $M_3$) receptor antagonist), other $\beta_2$-adrenoreceptor agonists, antiinfective agents (e.g. antibiotics, antivirals), or antihistamines for the preparation of the dry powder formulation. In a further embodiment of the invention, a combination comprising vilanterol or a pharmaceutically acceptable salt, solvate or physiologically functional derivative thereof, preferably vilanterol triphenylacetate, together with one or more other therapeutically active substance that is selected from a group comprising an anti-inflammatory agent (e.g. a corticosteroid or an NSAID), an anticholinergic agent, another $\beta_2$- adrenoreceptor agonist, an antiinfective agent (e. g. an antibiotic or an antiviral), or an antihistamine is used in the process of the invention as the active substance. Preferred are combinations comprising vilanterol or a pharmaceutically acceptable salt, solvate or physiologically functional derivative thereof, preferably vilanterol triphenylacetate, together with a corticosteroid selected from a group comprising mometasone, fluticasone, budesonide; and/or an anticholinergic selected from a group comprising tiotropium, oxitropium, glycopyrronium, ipratropium, aclidinium; and/or a PDE-4 inhibitor selected from a group comprising roflumilast, rolipram, ibudilast, cilomilast.

The other therapeutic substance(s) may be used in the form of salts, (e. g. as alkali metal or amine salts or as acid addition salts), or pro drugs, or as esters (e. g. lower alkyl esters), or as solvates (e. g. hydrates). It will be clear also that where appropriate, the therapeutic substances may be used in optically pure form.

The dry powder formulation prepared by the process of the present invention is used in the prophylaxis and treatment of clinical conditions for which a selective $\beta_2$-adrenoreceptor agonist is indicated. Such conditions include diseases associated with reversible airways obstruction such as asthma, chronic obstructive pulmonary diseases (COPD) (e. g. chronic and wheezy bronchitis, emphysema), respiratory tract infection and upper respiratory tract disease (e.g. rhinitis, including seasonal and allergic rhinitis).

The following example serves only to illustrate the present invention further without restricting its scope to the embodiments provided hereinafter by way of example.

**Example**

| Content of the formulation | Amount (%) |
|---|---|
| Vilanterol triphenylacetate | 0.1% - 1.0% |
| Lactose monohydrate | 85% - 99% |
| Magnesium stearate | 0.02% - 1.0% |
| The percentage amount range of each component (showed in the table) is calculated by weight based on the total amount of the dry powder formulation. | |

For the preparation of formulation in the table given above, initially the components of the formulation are weighted to the amount falling within the range that is showed in the above table for each component. If it is necessary, any of the components of the formulation is micronized in a microniser (e.g. air-jet mill micronizer) to obtain said component with desired volume median diameter defined in the description before the mixing process. Then, vilanterol triphenylacetate and the magnesium stearate are added alternately layer by layer to a suitable mixing apparatus while the lactose monohydrate is continuously metered into the same mixing apparatus concurrently. The mixing process during the preparation of the dry powder formulation is performed using a high shear mixer or a low shear tumbling mixer, whichever is appropriate, with the rate of 2 to 250 rpm.

**Claims**

1. The process for preparing the dry powder formulation **characterized in that** an active substance and an additive are added alternately layer by layer to a suitable mixing apparatus while a pharmaceutically acceptable carrier is continuously metered into the same mixing apparatus concurrently.

2. The process according to claim 1, wherein the active substance and the additive are divided into the same number of the equal-size portions separately.

3. The process according to claim 1 or claim 2, wherein the active substance and the additive are divided into at least two equal-size portions separately.

4. The process according to any of claim 1 to claim 3, wherein the additive is magnesium stearate.

5. The process according to any of the preceding claims, wherein the pharmaceutically acceptable carrier, the magnesium stearate and the active substance, are added through a suitable screening apparatus.

6. The process according to any of the preceding claims, wherein the pharmaceutically acceptable carrier is selected from the group comprising lactose, mannitol, glucose, trehalose, cellobiose, sorbitol, maltitol or a combination of two or more of them.

7. The process according to claim 6, wherein the pharmaceutically acceptable carrier is lactose.

8. The process according to claim 6 or claim 7, wherein the volume median diameter of lactose is between 30 $\mu$m and 250 $\mu$m.

9. The process according to any of the preceding claims, wherein the amount of the magnesium stearate is less than 1.5% by weight based on the total amount of the dry powder formulation.

**10.** The process according to claim 9, wherein the volume median diameter of magnesium stearate is between 1 $\mu$m and 100 $\mu$m.

**11.** The process according to any of the preceding claims, wherein the active substance is in an amount of 0.05% to 2.5% by weight based on the total amount of the dry powder formulation.

**12.** The process according to any of the preceding claims, wherein the active substance is vilanterol triphenylacetate.

**Patentansprüche**

**1.** Verfahren zum Bereiten einer Trockenpulverrezeptur, **dadurch gekennzeichnet, dass** eine aktive Substanz und ein Additiv alternierend Schicht um Schicht in eine geeignete Mischvorrichtung hinzugegeben werden, während gleichzeitig ein pharmazeutisch akzeptabler Träger kontinuierlich in dieselbe Mischvorrichtung dosiert wird.

**2.** Verfahren nach Anspruch 1, wobei die aktive Substanz und das Additiv getrennt voneinander in die gleiche Zahl von gleich großen Portionen unterteilt werden.

**3.** Verfahren nach Anspruch 1 oder Anspruch 2, wobei die aktive Substanz und das Additiv getrennt voneinander in wenigstens zwei gleich große Portionen unterteilt werden.

**4.** Verfahren nach einem der Ansprüche 1 bis 3, wobei das Additiv Magnesiumstearat ist.

**5.** Verfahren nach einem der vorstehenden Ansprüche, wobei der pharmazeutisch akzeptable Träger, das Magnesiumstearat und die aktive Substanz über eine geeignete Klassierungs- bzw. Siebvorrichtung hinzugegeben werden.

**6.** Verfahren nach einem der vorherstehenden Ansprüche, wobei der pharmazeutisch akzeptable Träger ausgewählt ist aus der Gruppe, die Laktose, Mannitol, Glucose, Trehalose, Cellobiose, Sorbitol, Maltitol beinhaltet, oder aus einer Kombination von zwei oder mehr hiervon.

**7.** Verfahren nach Anspruch 6, wobei der pharmazeutisch akzeptable Träger Laktose ist.

**8.** Verfahren nach Anspruch 6 oder Anspruch 7, wobei der mittlere Volumendurchmesser ("volume median diameter") von Laktose zwischen 30 $\mu$m und 250 $\mu$m liegt.

**9.** Verfahren nach einem der vorstehenden Ansprüche, wobei die Menge an Magnesiumstearat kleiner ist als 1,5 Gew.-%, und zwar basierend auf der Gesamtmenge der Trockenpulverrezeptur.

**10.** Verfahren nach Anspruch 9, wobei der mittlere Volumendurchmesser von Magensiumstearat zwischen einem 1 $\mu$m und 100 $\mu$m liegt.

**11.** Verfahren nach einem der vorstehenden Ansprüche, wobei die aktive Substanz in einer Menge von 0,05 Gew.-% bis 2,5 Gew.-% vorliegt, und zwar basierend auf der Gesamtmenge der Trockenpulverrezeptur.

**12.** Verfahren nach einem der vorstehenden Ansprüche, wobei die aktive Substanz Vilanteroltriphenylazetat ist.

**Revendications**

**1.** - Procédé de préparation de formulation de poudre sèche **caractérisé par le fait qu'**une substance active et un additif sont ajoutés de manière alternée couche par couche à un appareil de mélange approprié tandis qu'un vecteur pharmaceutiquement acceptable est dosé de manière continue dans le même appareil de mélange simultanément.

**2.** - Procédé selon la revendication 1, dans lequel la substance active et l'additif sont divisés en un nombre identique de parties de taille égale séparément.

**3.** - Procédé selon la revendication 1 ou la revendication 2, dans lequel la substance active et l'additif sont divisés en au moins deux parties de taille égale séparément.

**4.** - Procédé selon l'une quelconque des revendications 1 à 3, dans lequel l'additif est le stéarate de magnésium.

**5.** - Procédé selon l'une quelconque des revendications précédentes, dans lequel le vecteur pharmaceutiquement acceptable, le stéarate de magnésium et la substance active sont ajoutés par l'intermédiaire d'un appareil de criblage approprié.

**6.** - Procédé selon l'une quelconque des revendications précédentes, dans lequel le vecteur pharmaceutiquement acceptable est choisi parmi le groupe comprenant le lactose, le mannitol, le glucose, le tréhalose, le cellobiose, le sorbitol, le maltitol ou une combinaison d'au moins deux de ceux-ci.

**7.** - Procédé selon la revendication 6, dans lequel le vecteur pharmaceutiquement acceptable est le lactose.

**8.** - Procédé selon la revendication 6 ou la revendication 7, dans lequel le diamètre moyen en volume de lactose est entre 30 $\mu$m et 250 $\mu$m.

**9.** - Procédé selon l'une quelconque des revendications précédentes, dans lequel la quantité de stéarate de magnésium est de moins de 1,5 % en poids sur la base de la quantité totale de la formulation de poudre sèche.

**10.** - Procédé selon la revendication 9, dans lequel le diamètre moyen en volume de stéarate de magnésium est entre 1 $\mu$m et 100 $\mu$m.

**11.** - Procédé selon l'une quelconque des revendications précédentes, dans lequel la substance active est dans une quantité de 0,05 % à 2,5 % en poids sur la base de la quantité totale de la formulation de poudre sèche.

**12.** - Procédé selon l'une quelconque des revendications précédentes, dans lequel la substance active est vilanterol triphénylacétate.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03024439 A1